Europäisches Patentamt

European Patent Office

Office européen des brevets

(11) Publication number: **0 154 731**
**A1**

## (12) EUROPEAN PATENT APPLICATION

(21) Application number: 84201957.2

(51) Int. Cl.⁴: **A 61 F 2/16**

(22) Date of filing: 28.12.84

---

(30) Priority: 21.01.84 NL 8400200

(43) Date of publication of application: 18.09.85
Bulletin 85/38

(84) Designated Contracting States: **AT BE CH DE FR GB IT LI LU NL SE**

(71) Applicant: **Treffers, Willem Frits, Kerkstraat 18, NL-6585 AV Mook (NL)**

(72) Inventor: **Treffers, Willem Frits, Kerkstraat 18, NL-6585 AV Mook (NL)**

(74) Representative: **Noz, Franciscus Xaverius, Ir. et al, Boschdijk 155 P.O. Box 645, NL-5600 AP Eindhoven (NL)**

---

(54) Intraocular lens.

(57) An intraocular lens made at least partly from a hydrophilic soft lens material whereby the lens is free of a fastening or supporting member and has, in the non-hydrated state, a central thickness of at least 0.5 mm.

EP 0 154 731 A1

-1-

Intraocular lens.

The invention relates to an intraocular lens at least partly made from a hydrophilic, soft lens material. The invention more particularly relates to a method of implanting an intraocular lens by carrying out an extracapsular cataract extraction and by subsequently arranging an artificial lens in the lens capsule. As stated in "Lens implantation 30 years of progress" by P. Leonard and J. Rommel, issued by Dr. W. Junk Publishers, The Hague, Boston, London, 1982, the idea of replacing an opaque eye lens by an intraocular artificial lens dates from more than two hundred years and has to be attributed to two Italian surgeons Tadiny and Casaamata. In 1949 a new era for lens implantation started when Harold Ridley in London placed the first artificial lens. The small plastic lens was disposed at the place of the eye lens itself, that is to say, behind the pupil. The lenses then implanted were made from polymethyl methacrylate (PMMA), which material was accepted by the tissue, at least it was not repelled. This method was used on a limited scale, but after some years it was abandoned because the results obtained were very precarious. During the fifties a different kind of implant lenses was used, which consisted of an optical part made from PMMA and a part ensuring the stability of the fastening or supporting members made from Nylon, polypropylene, platinum or a different metal. These implant lenses were fixed in front of the pupil with the aid of two or three supporting members disposed between iris and cornea. The results of these implant lenses were not much better than those of the lenses first used so that in the beginning of the sixties it was assumed that lens implantation was not safe.

By further research the implant lenses have been further improved and the implant lenses used in the seventies may be divided into three groups dependent on the method of fixation, to wit: a) lenses supported by the iris, b) lenses supported by the original lens capsule and the iris and c) lenses supported in the chamber corner. The implant lenses a) and c) are placed after an intracapsular cataract extraction has been carried out, in which technique the lens contents together with the capsule are removed integrally, whereas the implant lenses of group b) are placed after an extracapsular cataract extraction has been performed, in which technique the lens capsule is opened and the contents are removed. Herein the remainder of the capsule and the zonulae remain intact so that they can be used for fixing an intraocular lens.

The invention will be further described with reference to the accompanying drawing, in which

Fig. 1 shows part of the eye,

Fig. 2 shows a lens implanted in the eye supported in the chamber corner,

Fig. 3 shows a lens disposed in the front chamber of the eye,

Fig. 4 shows a lens with fastening or supporting members to be arranged in the front chamber of the eye,

Fig. 5 shows an alternative embodiment of an intraocular lens of Fig. 4,

Fig. 6 shows a lens arranged in the rear chamber of the eye,

Fig. 7 shows the same lens as Fig. 6 but arranged in the front chamber,

Fig. 8 shows a lens differing from that of Fig. 6 arranged in the rear chamber of the eye,

Fig. 9 shows the lens as used in Fig. 8,

Fig. 10 shows the lens which can be used in accordance with the invention and

Fig. 11 shows a contact lens which can be externally applied to the cornea.

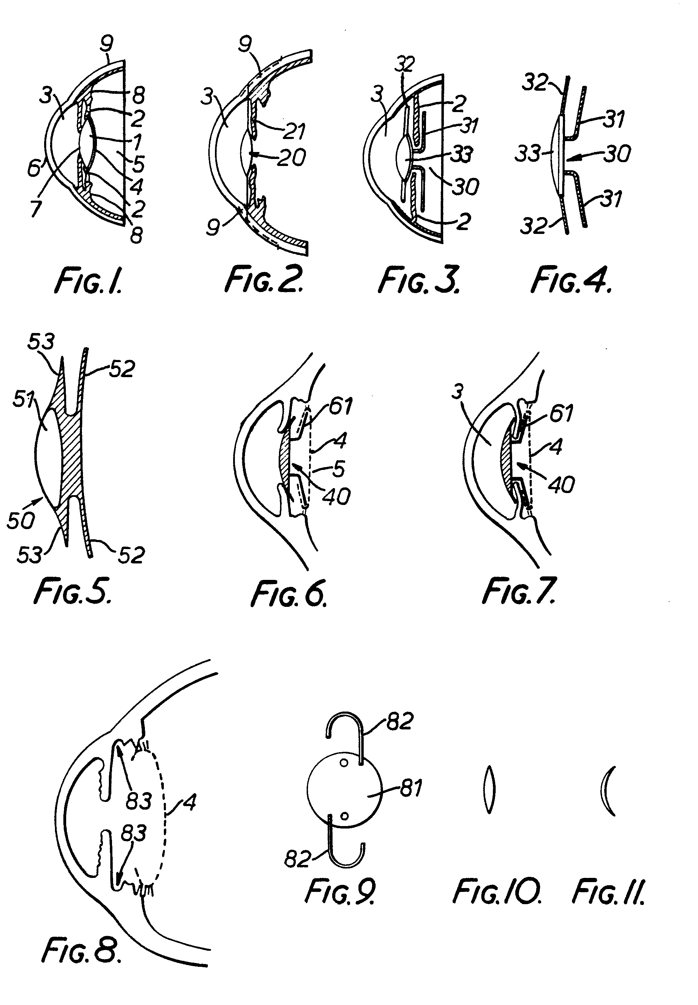

Fig. 1 shows part of the natural eye for a good understanding of the invention. The lens 1 is located behind the iris 2 and the anterior chamber 3. The lens 1 is accommodated in the lens capsule 4, which is located in the posterior chamber 5. The front side of the eye is formed by the cornea 6 and the pupil opening 7 between the iris parts 2 is treated. The eye lens 1 is held in place in the capsule 4, the capsule being connected with the aid of zonulae 8 forming the suspension tissue of the eye lens with the sclera 9, which is a tough leather skin. By means of the zonulae 8 the lens 1 has its accommodation capacity, which also can be reached according to the invention.

In the intracapsular cataract extraction the lens contents together with the capsule are integrally removed, whilst the zonulae 8 are torn. This extraction is easier and can be carried out with a more predictible result than the extra capsular cataract extraction in which the lens capsule is opened and the opaque contents are removed. The remainder of the capsule and the zonulae remain intact and can be used for fixing an intraocular lens.

Figs. 2, 3 and 7 show intraocular lenses arranged in the anterior chamber 3. These lenses 20, 30 and 40 are provided with supporting and fastening members.

Fig. 2 shows a lens 20 implanted in 1958 by Strampelli in ltaly (pages 11 and 12 of the book "Lens implantation" by P. Leonard and J. Rommel), in which the thin Nylon wires are arranged in the sclera 9.

Fig. 3 shows an intraocular lens 30 having an optical part 33 made from a hard lens material and having fastening or supporting members 31 and 32 clamped around the iris 2. The lens itself is shown for the sake of clarity in Fig. 4 and a similar lens is shown in Figs. 5, 6 and 9. With respect to these lenses much literature is available particularly because various intraocular lenses have been developed with different fastening members. Since 1960, there have become known the iris clip lens developed by Binkhorst, the Copland lens developed in America, the medallion  lens and the platinum clip lens developed in The Netherlands and in America and the "Sputnik" lens developed in Russia. Various embodiments of these lenses are mentioned in the U.S. Patents 4,085,467 and 4,134,160, in French Patent Application 2,239,234 and in Dutch Patent 171,120.

The optical part of the intraocular lens shown in Figs. 2 to 4 and 6 to 9 is made from a hard lens material, preferably polymethyl methacrylate. In contrast thereto an article of K.R. Mehta _et alia_ in Am. Intra-ocular Implant, Soc. J., Vol. IV, October 1978 has proposed to make the lens from poly-HEMA. The lens proposed by K.R. Mehta is shown in Fig. 5 and in the book of P. Leonard and J. Rommel this article is discussed on pages 242 and 243.

The lens 50 shown in Fig. 5 is made from a specific soft lens material i.e. Soflex 44-R, which can be hydrated for 40 %, whilst by the specific design of the fastening and supporting members 52 and 53 a reasonable fixation to the iris can be obtained. These lenses are implanted in both an extracapsular and an intracapsular manner. A disadvantage of this lens is that its firmness depends on the hydratation so that with a hydratation degree of more than 40 % the lens becomes looser in front of or behind the iris.

Figs. 6 and 7 show an intraocular lens 40 arranged in the posterior chamber 5 (Fig. 6) and in the anterior chamber 3 (Fig. 7). In both cases the lens capsule 4 is maintained and the fastening members 61 are placed in the lens capsule 4. A disadvantage of this extracapsular cataract extraction technique is that, although the lens capsule 4 is saved, this capsule starts wrinkling after some time. Moreover, the lens 40 arranged in the manner shown in Fig. 6 is less firmly fixed.

This technique has been developed by C.D. Binkhorst and mentioned in the book "Lens Implantation" on page 292.

In 1977 Shearing developed a lens of the kind shown in Fig. 9 consisting of a planoconvex optical part 81 and two loops 82 of polypropylene. The loops 82 are slightly resilient and thus can provide a clamping effect when after an extracapsular cataract extraction the lens is placed in the lens capsule 4 and/or in the sulcus 83 (Fig. 8).

Recapitulating it can be stated that the development in the domain of intraocular lenses after 1960 has resulted in lenses having an optical part of a hard lens material and provided with fastening or supporting members, in which case an intracapsular cataract extraction was carried out, which was relatively easily performable, but the capsule and the zonulae were torn or an extracapsular cataract extraction was carried out, which was relatively difficult, but the remainder of the capsule and the zonulae remained intact which capsule often started wrinkling due to the incomplete filling of the capsule wall.

By the experiments carried out a new type of lens has been developed in accordance with the invention; it is characterized in that the lens is free of fastening or supporting members and has, in a non-hydrated state, a central thickness of 0.5 mm at the least. The intraocular lens embodying the invention is, therefore as sated in the preamble, made at least partly from a hydrophilic, soft lens material. This lens can be completely made from a hydrophilic, soft lens material, and in the non-hydrated state this lens is placed in the lens capsule after an extracapsular cataract extraction. In the non-hydrated state the lens is of a hard, readily workable material of minimum dimensions. After hydratation the lens material will swell and the tendency is to cause the lens to assume the shape of the original lens after hydration so that the lens capsule 4 will be filled as completely as possible. Thus the disadvantage hitherto occurring that the capsule often starts wrinkling will no longer occur. By attracting moisture from the aqueous humor of the eye the intraocular lens will swell. By the accurate fit in the capsule bag the hydrated lens is fixed. Moreover, internal forces in the eye may produce via the natural adhesion of the zonulae 8, a deformation of the soft lens so that some accommodation capacity may be regained. This would be an improvement over the known intraocular lenses with fastening or supporting members because such an accommodation capacity is excluded in the known lenses.

The lens embodying the invention is essentially made from a soft lens material, which has so far also been used for making soft contact lenses or from a material still being developed for making soft contact lenses. Such contact lenses are specifically mentioned in U.S. Patent Specification 3,689,673. As compared with the soft contact lens the lens embodying the invention differs in that the soft contact lens is always used in the hydrated state, whereas the lens embodying the invention is hydrated only after implantation in the lens capsule. In the manufacture of the soft contact lens, in which the starting material is the hard, non-hydrated lens material, an intermediate product will be obtained during manufacture, which may exhibit some similarity to the intraocular lens in accordance with the invention, but the non-marketed semi-product of the contact lens will be thinner than the minimum thickness of the intraocular lens, which exceeds 0.5 mm. The mean thickness of a standard contact lens is 0.14 mm, whilst the ultra-thin lenses have a central thickness of 0.07 mm.

If the refraction of the lens has to be modified, the lens embodying the invention may have a core of a hard lens material such as polymethyl methacrylate and the coating of the core may be made from a soft lens material. At any rate the outer part of the lens has to be made from a soft, hydrophilic lens material because otherwise adaptation in the hydrated state cannot be obtained to the shape of the implanted lens. It is advantageous to implant the lens in the dehydrated state because then the lens has a minimum size and can be most readily manipulated. Preferably the shape of the lens embodying the invention is biconvex, whilst in accordance with the natural lens the lens has a weak front curve and a stronger rear curve, although the lens according to the invention is not limited thereto because it is a primary requirement for the lens to satisfactorily fit in the lens capsule, which may also be achieved with a non-biconvex lens.

The lens embodying the invention may lead to a new generation of intraocular lenses and to simplification of the treatment of cataract.

The ifgures used in the claims are only meant to explain more clearly the intention of the invention and are not supposed to be any restriction concerning the interpretation of the invention.

0154731

-1-

CLAIMS

1.      An intraocular lens made at least partly from a hydrophilic soft lens material characterized in that the lens is free of a fastening or supporting member and has, in the non-hydrated state, a central thickness of at least 0.5 mm.

2.      An intraocular lens as claimed in Claim 1 characterized in that the core is made from a hard lens material and the outer part from a soft lens material.

3.      An intraocular lens as claimed in Claims 1 and 2 characterized in that in the non-hydrated state the dimensions of the lens are chosen so that the non-hydrated lens can be readily placed in the lens capsule and that the hydrated lens fits in the lens capsule.

4.      An intraocular lens as claimed in Claims 1 to 3 characterized in that the lens (10) is biconvex and, after hydration, approaches the shape of the natural lens.

5.      An intraocular lens as claimed in Claims 1 to 4 characterized in that the lens is mainly made from polyhydroxy ethylmethacrylate (poly-HEMA).

6.      An intraocular lens as claimed in Claims 2 and 5 characterized in that the outer part is made from poly-HEMA and the core mainly from polymethyl methacrylate.

7.      A method of implanting an intraocular lens by carrying out an extracapsular cataract extraction and by subsequently implanting an artificial lens in the lens capsule characterized in that the artificial lens as defined in Claims 1 to 6 is implanted and is hydrated after implantation.

0154731

FIG.1.

FIG.2.

FIG.3.

FIG.4.

FIG.5.

FIG.6.

FIG.7.

FIG.8.

FIG.9.

FIG.10.

FIG.11.

# PARTIAL EUROPEAN SEARCH REPORT

European Patent Office

which under Rule 45 of the European Patent Convention shall be considered, for the purposes of subsequent proceedings, as the European search report

015 4731

Application number

EP 84 20 1957

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US - A - 4 254 509 (TENNANT) <br><br> * Figures; column 2, line 8 - column 3, line 19 * | 1,3,4,5 | A 61 F 2/16 |
| Y | US - A - 4 373 218 (SCHACHAR) <br><br> * The entire document * | 1,3,4,5 | |
| A | DE - A - 3 208 729 (KRUMEICH) <br><br> * Abstract; figures * | 1,3,5 | |
| A | US - A - 4 254 510 (TENNANT) <br><br> * Figures; claim 6 * <br><br> -- ./. | 1,2,5,6 | TECHNICAL FIELDS SEARCHED (Int. Cl.4) <br><br> A 61 F |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6
Claims searched incompletely:
Claims not searched: 7
Reason for the limitation of the search:

Intraocular lens. Method of inplanting an intraocular lens. Method for treatment of the human or animal body by surgery or therapy (see art. 52(4) of the European Patent Convention).

| Place of search | Date of completion of the search | Examiner |
|---|---|---|
| The Hague | 11-04-1985 | STEENBAKKER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

**PARTIAL EUROPEAN SEARCH REPORT**

0154731

Application number

EP 83 20 1957 -2-

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | US - A - 4 240 163 (GALIN) | | |
| A | NL - A - 78 11605 (AMERICAN HOSPITAL SUPPLY CORP.) | | |
| A | US - A - 4 409 691 (LEVY) | | |
| PX | US - A - 4 449 257 (KOENIGER) | | |
| | * Figures; abstract * | 1,3,4, 5 | |
| | ------ | | |

TECHNICAL FIELDS SEARCHED (Int. Cl.4)